Europäisches Patentamt

European Patent Office

Office européen des brevets

(11) Publication number: 0 272 492 B1

(12) EUROPEAN PATENT SPECIFICATION

(45) Date of publication of patent specification: 19.02.92  (51) Int. Cl.⁵: **A61M 35/00, A45D 37/00**

(21) Application number: **87117480.1**

(22) Date of filing: **26.11.87**

(54) **Applicator for liquid.**

(30) Priority: **13.12.86 JP 297320/86**
**27.04.87 JP 103732/87**
**19.05.87 JP 74821/87**

(43) Date of publication of application:
**29.06.88 Bulletin 88/26**

(45) Publication of the grant of the patent:
**19.02.92 Bulletin 92/08**

(84) Designated Contracting States:
**AT CH DE FR GB IT LI**

(56) References cited:
**DE-A- 2 328 177      FR-A- 1 394 228**
**FR-A- 2 346 243      GB-A- 1 062 333**
**US-A- 2 999 265      US-A- 3 082 468**
**US-A- 3 124 825      US-A- 3 369 267**
**US-A- 3 625 351      US-A- 3 980 224**
**US-A- 4 427 115**

(73) Proprietor: **LEAD CHEMICAL COMPANY LTD.**
**77-3, Mimata Toyama-shi**
**Toyama-ken(JP)**

(72) Inventor: **Mori, Masao**
**248, Tenshoji**
**Toyama-shi Toyama-ken(JP)**
Inventor: **Yokoi, Hideharu**
**39-3, Yokohouonji Kamiichimachi**
**Nakanikawa-gun Toyama-ken(JP)**

(74) Representative: **Reinhard, Skuhra, Weise**
**Friedrichstrasse 31**
**W-8000 München 40(DE)**

EP 0 272 492 B1

## Description

### Background of the Invention

#### 1. Field of the Invention

The present invention relates to a disposable applicator for liquid according to the preamble of claim 1. Particularly, it is concerned with handy and disposable applicators for liquid such as liquid drugs for percutaneous application like analgesics, drugs for athlete's foot, anti-urticants, sterilizers and antiseptics, hair tonics, drugs for suppurative diseases, eye lotions, anti-hemorrhoidal agents, stomatic drugs, or the like; or cosmetic liquid such as manicure removers, etc.

#### 2. Description of the Prior Art

As liquid drugs for percutaneous application, for example, analgesics, a variety of preparations such as spray type or cheer type have been hitherto used widely. These preparations are excellent in their quick effects but the volume is approximately 100 ml so that the preparations involve disadvantages that they are inconvenient for storage and are not handy. Further with respect to drugs for athlete's foot, repeated use of applicators are unsuitable because of sterilization and contamination by dusts, etc.

In recent years, applicators for liquid drugs which are convenient for storage and handy have been proposed. As examples of these applicators, mention may be made of those disclosed in Published Examined Japanese Utility Model Application No. 38527/85, Published Unexamined Japanese Utility Model Application Nos. 18188/79 and 163050/80, etc. An applicator according to the preamble of claim 1 is disclosed in US-A-3 124 825. disclosed in US-A-3 124 82

However, the applicators for liquid drugs described above are not sufficiently satisfactory because they have complicated structures or are difficult to use.

### Summary of the Invention

An object of the present invention is to provide disposable and handy applicators for liquid which improve defects of the above-described conventional containers or applicators for liquid or liquid drugs applied percutaneously, and are convenient for storage, handy and disposable in every use.

Namely, the disposable applicator for liquid of the present invention is characterized by the features of the characterizing portion of claim 1. Further advantageous embodiments are specified in the subclaims.

### Brief Description of the Drawings

Figure 1 is a plane view of the disposable applicator for liquid of the present invention shown in Example 1.

Figure 2 is a plane view showing a state where the applicator for liquid shown in Figure 1 is opened.

Figure 3 is a plane view showing a state where the holding member is pushed out of the applicator for liquid of Figure 2.

Figure 4 is a perspective view of Figure 3.

Figure 5 is a perspective view showing a state where a liquid drug is applied to the skin of human using the applicator for liquid shown in Figure 1.

Figures 6 and 7 are plane views of the applicators for liquid of the present invention in Examples 2 and 3.

Figure 8 is a plane view of the disposable applicator for liquid of the present invention shown in Example 4.

Figure 9 is a cross sectional view of the applicator shown in Figure 8 along A-A' line.

### Detailed Description of the Invention

Preferred embodiments of the disposable applicator for liquid of the present invention include those described below:

(i) A disposable applicator for liquid wherein a slit is provided in the laminate bag by cutting a part of the laminate bag so as to expose a part of the holding member or so as to push a part of the holding member out of an opening.

(ii) A disposable applicator for liquid described in (i) above wherein a part of the holding member is engaged with the opening and the slit is provided so as not to push the whole of the holding member out of the opening.

(iii) A disposable applicator for liquid wherein the laminate bag is of stick wrapping type.

(iv) A disposable applicator for liquid described in (iii) above wherein the back surface of one end along the length direction is adhered to the surface of another end in stick wrapping.

(v) A disposable applicator for liquid described in (iv) above wherein the end having the back surface adhered has a margin being free of the holding member, and at least one slit is provided perpendicular to the length direction of the margin.

(vi) A disposable applicator for liquid described in (iii) above wherein the back surface of one end along the length direction is adhered to the back surface of another end in stick wrapping.

(vii) A disposable applicator for liquid described in (vi) above wherein at least one slit is provided on the adhered portion along the length direction.

(viii) A disposable applicator for liquid described in (vi) above wherein the laminate bag comprises a laminate of polyester, aluminum and polyethylene.

(ix) A disposable applicator for liquid wherein the laminate bag comprises a laminate of polyethylene, aluminum, a monoaxially stretched film of crystalline polymer and polyethylene.

(x) A disposable applicator for liquid wherein a perforation is provided in the outermost layer of the laminate bag in a linear line to be opened.

(xi) A disposable applicator for liquid wherein the laminate bag comprises a laminate of monoaxially strongly stretched film of crystalline polymer and has a directional property of tearing resistance.

(xii) A disposable applicator for liquid wherein the laminate bag comprises a dry laminate film.

(xiii) A disposable applicator for liquid wherein the holding member is a porous member comprising a woven cloth or an unwoven cloth of synthetic fibers or natural fibers such as rayon, polyester, polypropylene, polyamide, cotton, etc. or a synthetic or natural polymer such as a urethane foam, cellulose sponge, etc.

(xiv) A disposable applicator for liquid which is applied to human percutaneously.

(xv) A disposable applicator for liquid wherein the effective ingredient is a drug applied to human percutaneously an analgesic, a drug for athlete's foot, an anti-urticant, a sterilizer and antiseptic, a hair tonic, a drug for suppurative diseases, an eye lotion, an anti-hemorrhoidal agent, a stomatic drug or the like; or a cosmetic liquid such as a manicure remover, etc.

The wrapping mode of the laminate bag may be sealed at the three faces or four faces or may be of stick wrapping.

In the stick wrapping, the back surface of one end may be adhered to the surface of another end, along the length direction (envelope adhesion); alternatively, the back surface of one end may also be adhered to the back surface of another end (putting-hands-together adhesion).

The adhesion may be effected in a conventional manner, for example, by means of thermal adhesion or adhesion with adhesives.

By providing a desired number spaced perforations perpendicular to the length direction of the margin, the laminate bag can be opened upon use.

As the laminate film, it is necessary to use materials having a strong impact strength, chemical resistance, low permeability and adsorption of ingredients and free from stripping of the laminate film between layers by chemicals or solvents. Preferred examples of the laminate film used for putting-hands-together adhesion type include combinations of polyester, 12 $\mu$m/aluminum, 7 $\mu$m/polyacrylonitrile film 20 $\mu$m, polyester, 12 $\mu$m/aluminum, 10 $\mu$m/polyethylene, 30 $\mu$m, etc. Further these layers are most suited for dry adhesion between the layers. It is required that the thickness of aluminum be 7 $\mu$m or more in view of generation of pinholes and strength.

Further when the laminate film provided with a perforation in the case where the bag is formed (for example, HAIGARU FILM manufactured by Okada Shigyo Co., Ltd., Japan) is used, the bag can be opened extremely easily.

By making a notch at the slit, opening of the bag can be made easy. Further by laminating monoaxially strongly stretched film of crystalline polymers, a linear opening becomes possible.

It is required for properties of the holding member that are resistant to chemicals and porous, have an excellent ability of adsorbing liquid and are capable of readily releasing the liquid by compression. Accordingly, it is preferred to use materials having high elasticity, for example, unwoven rayon cloth, polyurethane foam, etc.; for example, unwoven rayon cloth having a weight of 150 g/m$^2$ and a thickness of 7 mm can absorb approximately 8 to 10 ml of liquid per 1 g. In addition thereto, a woven cloth or an unwoven cloth of synthetic fibers or natural fibers such as rayon, polyester, polypropylene, polyamide, cotton, etc. or a synthetic or natural polymer such as a urethane foam, cellulose sponge, etc. can be used. Unwoven rayon cloth and polyurethane foam are particularly excellent in view of thermal adhesion with the laminate film.

The shape of holding member may be in that the cross section along the length direction can be various shapes, for example, a rectangle, a trapezoid, a triangle or combination thereof, etc. In order to engage a part of the holding member with the opening portion upon use, the holding member having a shape in which the width becomes broader along the pushing out direction, for example, the holding member of a trapezoid or a triangle, or the holding member having a shape in which the end of the pushing out direction projects in the width direction is used.

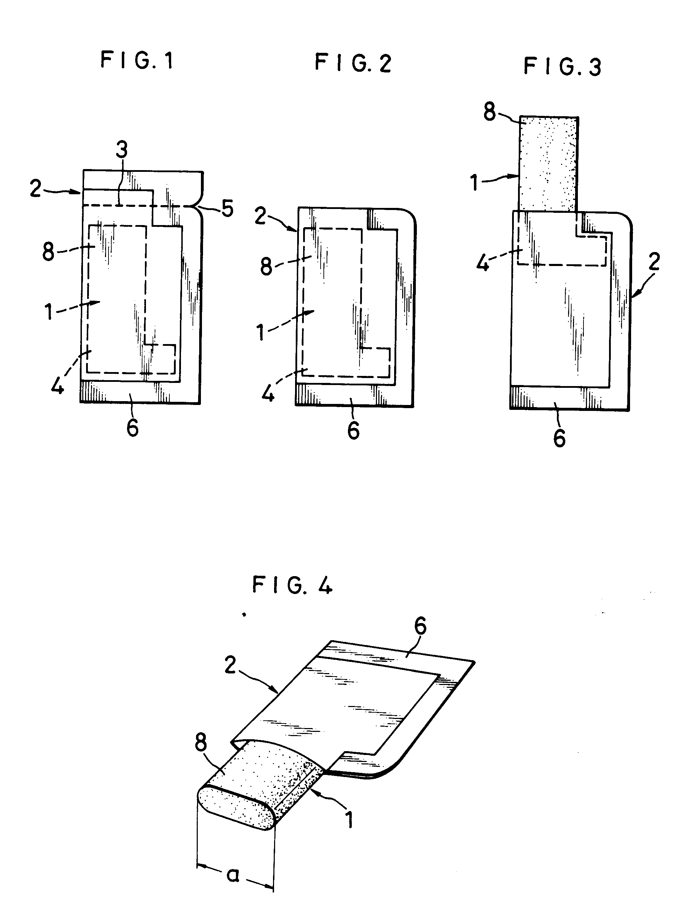

The size of the holding member varies depending upon area of affected portions varied depending upon kind of diseases; giving an example, in the case of the holding member having a shape as shown in Figure 4, it is preferred that the size (a) of the application part 8 be 15 to 22 mm with an analgesic, 5 to 10 mm with an agent for athlete's food, 10 to 20 mm with an anti-urticant and 10 to 15 mm with a manicure remover.

The present invention will be described in more detail with reference to the examples below but is not deemed to be limited thereto.

Example 1

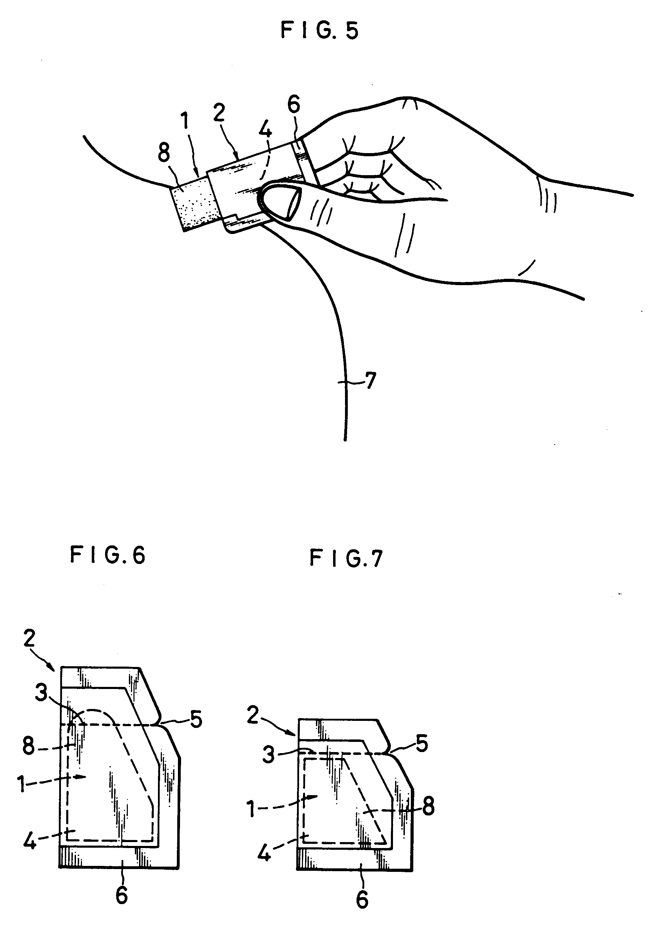

An example of the applicator for liquid of the present invention will be described concretely by referring to the drawings. A holding member 1 having a porous and elastic property is inserted in a laminate bag 2 shown in Figure 1. By packing liquid obtained by dissolving or dispersing effective ingredients in solvents such as water, alcohol, etc., the liquid is absorbed in holding member 1 and the laminate bag 2 is sealed. Numeral 6 indicates a thermally fused portion. Upon use, as shown in Figure 2, the upper edge portion of the laminate bag 2 is cut out of the slit 5 to open the bag. A perforation 3 is provided in the outermost layer of the laminate bag 2 so that the bag can be opened easily. Then, as shown in Figure 3, the holding member 1 is pushed out to expose an application part 8. In this case, the holding member 1 is engaged with the opening by the projection at the lower end of the holding member 1 and stays there. The length of the application part 8 can be freely controlled up to the distance when the holding member 1 is engaged. Figure 4 shows a perspective view of the state shown in Figure 3. Then, as shown in Figure 5, the lower end of the laminate bag 2 is picked up with the finger and moved along the affected region of the skin 7 at the application part 8, whereby the liquid drug is uniformly applied onto the affected region. In this case, a pressure portion 4 is pressed by the finger, whereby the liquid drug adsorbed in the interior of the holding member 1 is gradually exuded onto the application part 8. The applicator for liquid after use can be thrown away as it is.

Examples 2 and 3

In Figure 6 and 7, the applicators for liquid of the present invention of Examples 2 and 3 are shown. A holding member 1 of temple bell shape and a holding member 1 of trapezoidal shape are used in Figures 6 and 7, respectively. The holding members 1 in Figure 6 and Figure 7 are engaged with the opening portion of the laminate bag 2 at the lower ends thereof so that the whole of the bag is not pushed out.

Example 4

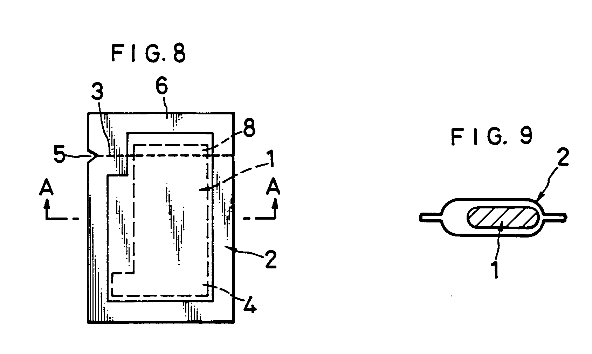

Figure 8 shows an example in which a laminate bag 2 having a shape sealed at the 4 faces thereof is used. Further Figure 9 shows a cross sectional view along A-A line of Figure 8.

Preparation Examples

Preparation examples which are used for the applicator for liquid of the present invention will be given below.

Preparation Example 1

A sterilizer and antiseptic was obtained by dissolving 30 mg of benzalkonium chloride, 100 mg of dibucaine hydrochloride, 50 mg of naphazoline hydrochloride and 250 mg of diphenhydramine hydrochloride in 100 ml of purified water.

Preparation Example 2

An analgesic for relieving skeletal muscle pains was obtained by dissolving 5 g of methyl salicylate, 5 g of d1-camphor, 5.5 g of 1-menthol, 6 ml of tincture of the Japanese Pharmacopeia, 100 mg of glycyrrhetinic acid and 100 mg of chlorpheniramine maleate in 70 ml of denaturated alcohol and adding purified water to the solution to make the whole 100 ml.

Preparation Example 3

An insect repellent was obtained dissolving 1 g of diphenhydramine hydrochloride, 10 ml of 10% ammonia water of the Japanese Pharmacopeia, 1.5 g of d1-camphor, 1 g of 1-menthol and 0.3 g of dibucaine hydrochloride in 50 ml of denaturated alcohol and adding purified water to the solution to make the whole 100 ml.

Examples of the holding member

Examples of various holding members used for the applicator for liquid in accordance with the present invention are shown below.
(1) Unwoven cloth made of rayon having a weight of 150 $g/m^2$ and cut into a thickness of 7 mm, a width of 20 mm and a length of 15 mm.
(2) Polyurethane foam having a porosity of 89%, and cut into a thickness of 7 mm, a width of 15 mm and a length of 10 mm.
(3) Unwoven cloth made of rayon having a weight of 150 $g/m^2$ and cut into a thickness of 5 mm, a width of 5 mm and a length of 10 mm.

Examples of the laminate bag

Examples of various laminate bags used for the applicator for liquid in accordance with the present invention are shown below.
(1) A bag of a laminate film comprising polyethylene, 30 $\mu$m/polyester, 12 $\mu$m/aluminum, 7 $\mu$m/polyethylene, 30 $\mu$m.
(2) A bag of a laminate film comprising polyester, 12 $\mu$m/aluminum, 7 $\mu$m/polyethylene, 30 $\mu$m.

(3) A bag of a laminate film comprising polyester, 12 µm/aluminum, 7 µm/polyacrylonitrile film, 20 µm.

(4) A bag of a laminate film comprising polyester, 12 µm/aluminum, 10 µm/polyacrylonitrile film, 20 µm.

The disposable applicator for liquid of the present invention has the construction described above and is, therefore, applicable to medicines, cosmetics, and the like for percutaneous application, for example, drugs for percutaneous or subcutaneous application such as analgesics for stiffness of the shoulders, waist pains, skeletal muscle pains, etc.; drugs for athlete's food, anti-urticants, sterilizers and antiseptics, hair tonics, drugs for suppurative diseases, eye lotions, anti-hemorrhoidal agents, stomatic drugs, or the like; or cosmetic liquid such as manicure removers, etc. Thus, the applicator for liquid can provide disposable medical products for percutaneous application. Further because the applicator for liquid is small-sized, lightweighted and inexpensive, the applicator for liquid is also useful as a handy drug in travelling.

Furthermore various modifications can also be made on the applicator for liquid of the present invention and therefore, most suitable modes can be chosen depending upon kind of effective ingredients, part to be applied, etc.

## Claims

1. A disposable applicator for liquid comprising a holding member (1) having a porous and elastic property in which liquid containing an effective ingredient is absorbed is movably sealed in a laminate bag (2), wherein means for providing a slit (5) is provided in said laminate bag (2) so as a part of said holding member (1) can be pushed out of an opening,
**characterized in that**
said holding member (1) has a larger cross-section than the opening and said slit (5) is such so as to prevent the whole of said member (1) being pushed out of the opening.

2. A disposable applicator for liquid as claimed in claim 1, wherein said laminate bag (2) is of stick wrapping type.

3. A disposable applicator for liquid as claimed in claim 2, wherein the back surface of one end along the length direction is adhered to the surface of the other end in stick wrapping.

4. A disposable applicator for liquid as claimed in claim 3, wherein the end having the back surface adhered has a margin being free of the holding member (1) and wherein at least one means for providing a slit (5) is provided perpendicular to the length direction of the adhered portion.

5. A disposable applicator for liquid as claimed in claim 2, wherein the back surface of one end along the length direction is adhered to the back surface of another end.

6. A disposable applicator for liquid as claimed in claim 5, wherein at least one means for providing a slit (5) is provided perpendicular to the length direction of the adhered portion.

7. A disposable applicator for liquid as claimed in claim 5, wherein said laminate bag (2) comprises a laminate of polyester, aluminum and polyethylene.

8. A disposable applicator for liquid as claimed in claim 1, wherein said laminate bag (2) comprises a laminate of polyethylene, aluminum, a monoaxially stretched film of crystalline polymer and polyethylene.

9. A disposable applicator for liquid as claimed in claim 1, wherein said laminate bag (2) comprises a laminate of monoaxially strongly stretched film of crystalline polymer and has a directional property of tearing resistance.

10. A disposable applicator for liquid as claimed in claim 1, wherein said laminate bag (2) comprises a laminate film.

11. A disposable applicator for liquid as claimed in claim 1, wherein said holding member (1) is a porous member comprising a woven cloth or an unwoven cloth of synthetic fibers or natural fibers such as rayon, polyester, polypropylene, polyamide, cotton, etc. or a synthetic or natural polymer such as a urethane foam, cellulose sponge.

12. A disposable applicator for liquid as claimed in claim 1, which is applied to human percutaneously.

13. A disposable applicator for liquid as claimed in claim 1, wherein said effective ingredient is a drug applied to human percutaneously an analgesic, a drug for athlete's foot, an anti-urticant, a sterilizer and antiseptic, a hair tonic, a drug for suppurative diseases, an eye lotion, an anti-hemorrhoidal agent, a stomatic drug or the like; or a cosmetic liquid such as a manicure remover.

## Revendications

1. Applicateur jetable pour liquide, comprenant un élément formant réservoir (1) qui a des propriétés poreuses et élastiques et qui est imprégné d'un liquide contenant un ingrédient actif, cet élément formant réservoir (1) étant enfermé hermétiquement, mais de façon à pouvoir se déplacer, dans un sachet (2) en produit stratifié, des moyens permettant de réaliser une fente (5) étant prévus dans ce sachet (2) en produit stratifié, de telle façon qu'une partie de l'élément formant réservoir (1) puisse être repoussée à l'extérieur d'une ouverture,
   caractérisé en ce que
   l'élément formant réservoir (1) a une section transversale supérieure à celle de l'ouverture et en ce que la fente (5) est telle qu'elle empêche la totalité de l'élément (1) d'être repoussée hors de l'ouverture.

2. Applicateur jetable pour liquide suivant la revendication 1, dans lequel le sachet (2) en produit stratifié est du type enveloppe de bâtonnet.

3. Applicateur jetable pour liquide suivant la revendication 2, dans lequel la surface arrière de l'une des extrémités suivant la direction longitudinale est collée sur la surface de l'autre extrémité de l'enveloppe de bâtonnet.

4. Applicateur jetable pour liquide suivant la revendication 3, dans lequel l'extrémité dont la surface arrière est collée possède un bord qui est dégagé de l'élément formant réservoir (1), et dans lequel au moins un moyen permettant de réaliser une fente (5) est prévu perpendiculairement à la direction longitudinale de la partie collée.

5. Applicateur jetable pour liquide suivant la revendication 2, dans lequel la surface arrière de l'une des extrémités suivant la direction longitudinale est collée sur la surface arrière d'une autre extrémité.

6. Applicateur jetable pour liquide suivant la revendication 5, dans lequel au moins un moyen permettant de réaliser une fente (5) est prévu perpendiculairement à la direction longitudinale de la partie collée.

7. Applicateur jetable pour liquide suivant la revendication 5, dans lequel le sachet (2) en produit stratifié est constitué d'un stratifié de polyester, d'aluminium et de polyéthylène.

8. Applicateur jetable pour liquide suivant la revendication 1, dans lequel le sachet (2) en produit stratifié est constitué d'un stratifié de polyéthylène, d'aluminium, d'un film de polymère cristallin à étirage monoaxial et de polyéthylène.

9. Applicateur jetable pour liquide suivant la revendication 1, dans lequel le sachet (2) en produit stratifié est constitué d'un stratifié de film de polymère cristallin à fort étirage monoaxial et possède une propriété directionnelle de résistance à la déchirure.

10. Applicateur jetable pour liquide suivant la revendication 1, dans lequel le sachet (2) en produit stratifié est constitué d'un film stratifié.

11. Applicateur jetable pour liquide suivant la revendication 1, dans lequel l'élément formant réservoir (1) est un élément poreux constitué d'un tissu tissé ou d'un tissu non tissé, en fibres synthétiques ou en fibres naturelles, telles que rayonne, polyester, polypropylène, polyamide, coton, etc., ou d'un polymère synthétique ou naturel, tel qu'une mousse d'uréthanne ou une éponge cellulosique.

12. Applicateur jetable pour liquide suivant la revendication 1, qui est appliqué sur l'être humain par voie percutanée.

13. Applicateur jetable pour liquide suivant la revendication 1, dans lequel l'ingrédient actif est un médicament appliqué à l'être humain par voie percutanée, un analgésique, un médicament pour les pieds des athlètes, un anti-urticant, un agent stérilisant et antiseptique, une lotion capillaire tonique, un médicament pour les maladies suppurantes, une lotion oculaire, un agent anti-hémorroïdal, un médicament gastrique ou analogue ; ou un liquide cosmétique tel qu'un dissolvant pour manucure.

## Patentansprüche

1. Einweggerät zum Auftragen von Flüssigkeit, bestehend aus einem porösen und elastischen Aufnahmeteil (1), in dem eine einen wirksamen Bestandteil enthaltene Flüssigkeit absorbiert ist, das bewegbar in einem laminierten Beutel (2) eingesiegelt ist, wobei eine Einrichtung zur Schaffung eines Schlitzes (5) in dem laminierten Beutel (2) derart vorgesehen ist, daß ein Abschnitt des Aufnahmeteils (1) aus einer Öffnung drückbar ist,
   **dadurch gekennzeichnet,**

daß das Aufnahmeteil (1) einen größeren Querschnitt als die Öffnung besitzt, und daß der Schlitz (5) derart bemessen ist, daß er das vollständige Herausdrücken des Aufnahmeteils (1) aus der Öffnung verhindert.

2. Einweggerät zum Auftragen von Flüssigkeit nach Anspruch 1, dadurch gekennzeichnet, daß der laminierte Beutel (2) nach Art einer Stickhülle ausgebildet ist.

3. Einweggerät zum Auftragen von Flüssigkeit nach Anspruch 2, dadurch gekennzeichnet, daß die Rückseite eines Endes entlang der Längsrichtung an der Fläche des anderen Endes der Stickhülle angeheftet ist.

4. Einweggerät zum Aufbringen von Flüssigkeit nach Anspruch 3, dadurch gekennzeichnet, daß das Ende, dessen Rückfläche angeheftet ist, einen Rand besitzt, bei dem sich das Aufnahmeteil (1) nicht befindet, und daß wenigstens eine Einrichtung zur Schaffung eines Schlitzes (5) senkrecht zu der Längsrichtung des angehefteten Abschnitts vorgesehen ist.

5. Einweggerät zum Aufbringen von Flüssigkeit nach Anspruch 2, dadurch gekennzeichnet, daß die Rückseite eines Endes entlang der Längsrichtung an der Rückseite des anderen Endes angeheftet ist.

6. Einweggerät zum Aufbringen von Flüssigkeit nach Anspruch 5, dadurch gekennzeichnet, daß wenigstens eine Einrichtung zur Schaffung eines Schlitzes (5) senkrecht zu der Längsrichtung des angehefteten Abschnitts vorgesehen ist.

7. Einweggerät zum Aufbringen von Flüssigkeit nach Anspruch 5, dadurch gekennzeichnet, daß der laminierte Beutel (2) ein Laminat aus Polyester, Aluminium und Polyethylen aufweist.

8. Einweggerät zum Aufbringen von Flüssigkeit nach Anspruch 1, dadurch gekennzeichnet, daß der laminierte Beutel (2) ein Laminat aus Polyethylen, Aluminium, einer monoaxial gestreckten Folie aus Kristallinpolymer und Polyethylen aufweist.

9. Einweggeärt zum Auftragen von Flüssigkeit nach Anspruch 1, dadurch gekennzeichnet, daß der laminierte Beutel (2) ein Laminat aus stark monoaxial gedehnter Folie von Kristallinpolymer ist und einen richtungsabhängigen Reißwiderstand besitzt.

10. Einweggerät zum Auftragen von Flüssigkeit nach Anspruch 1, dadurch gekennzeichnet, daß der laminierte Beutel (2) eine Laminatfolie aufweist.

11. Einweggerät zum Auftragen von Flüssigkeit nach Anspruch 1, dadurch gekennzeichnet, daß das Aufnahmeteil (1) ein poröses Teil ist, das einen gewebten Stoff oder einen ungewebten Stoff aus synthetischen Fasern oder Naturfasern, wie beispielsweise Rayon, Polyester, Polypropylen, Polyamid, Baumwolle, etc. oder ein synthetisches oder natürliches Polymer wie beispielsweise Urethanschaum, Zelluloseschaumstoff, aufweist.

12. Einweggerät zum Auftragen von Flüssigkeit nach Anspruch 1, zur perkutanen menschlichen Aufbringung.

13. Einweggerät zum Auftragen von Flüssigkeit nach Anspruch 1, dadurch gekennzeichnet, daß der wirksame Zusatz ein Medikament, das einem Menschen perkutan verabreicht wird, ein schmerzstillendes Mittel, ein Medikament gegen Fußpilz, ein Anti-Urtikant, ein Sterilisiermittel und ein Antiseptikum, ein Haartonik, eine Medizin für suppurative Krankheiten, eine Augenlotion, ein Mittel gegen Hämorrhoiden, ein Magenmittel oder dergleichen, oder eine kosmetische Flüssigkeit, wie beispielsweise Maniküreentferner ist.

FIG. 1

FIG. 2

FIG. 3

FIG. 4

8

# FIG. 5

# FIG.6

# FIG.7

FIG. 8

FIG. 9